# EUROPEAN PATENT SPECIFICATION

(11) **EP 0 705 934 B1**
(45) Date of publication and mention of the grant of the patent: **13.09.2000**
(21) Application number: 95119511.4
(22) Date of filing: 16.10.1991
(51) Int. Cl.: D04H 1/46, A61F 13/15, C08L 33/02

(54) **Environmentally friendly polymeric compositions and application of same**
Umweltfreundliche Polymerzusammensetzungen sowie Anwendungen desselben
Compositions polymériques non polluantes et leur application

(30) Priority: 16.10.1990 US 598276; 16.10.1990 US 598277
(43) Date of publication of application: 10.04.1996
(62) Divisional of application: 91117681.6
(73) Proprietor: KIMBERLY-CLARK WORLDWIDE, INC., Neenah, Wisconsin 54956 (US)
(72) Inventor: Durrance, Debra Hartley, Appleton, Wisconsin 54915 (US); Sasse, Philip A., Alpharetta, Georgia 30202 (US)
(74) Representative: DIEHL GLAESER HILTL & PARTNER

(56) References cited:
- US-A- 3 441 430
- US-A- 4 476 271
- DATABASE WPI Section Ch, Week 8340 Derwent Publications Ltd., London, GB; Class A82, AN 83-781778 XP002025369 & SU 979 441 A (ZAITSEVA N A) , 7 December 1982

## Description

In the production of personal care products, a number of different components and materials are required to construct the products. In the case of diaper manufacture, for example, these components include a backing material, which is a film, and an inner liner, which is typically a nonwoven web. Also, composite structures of synthetic and natural fibers have utility as absorbent media in a variety of personal care products. These various synthetic components are typically made from thermoplastic polymers such as polyethylene or polypropylene. However, with a greater emphasis being placed on protecting the environment today, there is a need to develop materials which are more compatible with existing and developing waste disposal technologies while still delivering the performance consumers have come to expect.

US-A-4 476,271 discloses an aqueous dispersion type thermosetting coating composition comprising 50-70 parts by weight of aqueous medium, 50-30 parts by weight of thermosetting powdery resin particles having an average particle diameter of 5-50µ and softening point of 30°-120°C, and 0.5-10 parts by weight of polyethylene glycol having an average molecular weight of 200-1000. The thermosetting resin particles are prepared by crosslinking of e.g. copolymers of (meth)acrylic acid and esters of (meth)acrylic acid.

US-A 3 441,230 discloses an adhesive consisting essentially of 100 pans by weight of a copolymer formed from 60 to 95 parts by weight of acrylic or methacrylic acid monomer and from 40 to 5 parts by weight of a water-soluble ether ester of acrylic or methacrylic acid, and 100 to 200 parts of compatible liquid water-soluble plasticizer having at least one ether linkage and a molecular weight above about 200.

Copolymers of (meth)acrylate esters and (meth)acrylic acid are of environmental interest because of their solubility in alkaline solutions or upon prolonged exposure to moisture, even though they are relatively hydrophobic. Unfortunately, the physical properties which make these materials desirable from an environmental standpoint can make them unsuitable for personal care products. In particular, films made from these polymers in contact with synthetic urine for a period greater than one hour will become hydrated, weak and sticky. This is obviously unacceptable for use in diapers, for example. Films made from these polymers also suffer from a lack of toughness and tear resistance. Films and nonwovens made from these copolymers are somewhat sticky and tend to stick or "block" in roll form. In addition, dimensional stability and aesthetic properties of these materials are also poor.

Therefore there is a need for copolymers of (meth)acrylate esters and (meth)acrylic acid which have modified properties suitable for use as components in personal care products.

The invention provides an absorbent article according to independent claim 1. Further advantageous features, aspects and details of the invention are evident from the dependent claims, the description, and the examples.

It has now been found that copolymers of (meth)acrylate esters and/or (meth)acrylic acid can be modified with additional materials to provide polymeric webs having improved properties suitable for use in personal care products. More specifically, it has been discovered that copolymers of ethylene and (meth)acrylic acid, when blended with these ester/acid copolymers, impart increased resistance to moisture for webs made therefrom. In addition, the tackiness of such webs is decreased, and dimensional stability and hand are markedly improved.

Furthermore, it has been discovered that polyethylene glycol, when blended with such ester/acid copolymers, imparts reduced viscosity and increased processability in meltblown and similar processes. In addition, the webs produced have a measure of elasticity and can be stretched with recovery. Furthermore, said polymeric webs can be heat treated to induce a reaction between the blended polymers, thus further alerting the characteristics of the web.

Hence in one aspect, the invention resides in an absorbent article comprising a nonwoven web or film prepared from a composition of matter comprising a polymeric blend of from about 60 to about 95 weight percent of a (meth)acrylate ester/(meth)acrylic acid copolymer and from about 5 to about 30 weight percent polyethylene glycol; wherein crosslinked copolymers of a (meth)acrylate ester and (meth)acrylic acid are excluded.

The relative proportions of the two polymers will depend upon the desired properties of the product into which they are to be made. For use in making nonwoven webs, such as meltblown webs, it is preferred that the copolymer blend contain from about 70 to about 90 weight percent of the ethyl acrylate/methacrylic acid copolymer and from about 5 to about 20 weight percent of the polyethylene glycol.

The absorbent article of the present invention has an outer cover, an absorbent core, and an inner liner, wherein any or all of said outer cover, absorbent core and inner liner comprise the webs (nonwoven or film) described herein. Such absorbent articles particularly include diapers and sanitary napkins.

The ethyl acrylate/methacrylic acid copolymer that is most preferred has a 4:1 ratio of the two comonomers by weight. The weight average molecular weight is about 150,000, with a melt flow rate of about 7 grams per 10 minutes, as measured at 170°C using a 2160 g weight and a 2.1 mm by 8 mm capillary. Clearly, however, many similar copolymers can be prepared that will provide similar attributes and can be substituted for the most preferred copolymer in these compositions. For example, any other (meth)acrylate ester derived from an alcohol having from 1 to 18 carbon atoms can be substituted for all or part of the ethyl acrylate. Such substitutions can lead to enhancement of particular properties for specific material applications. The manufacture of such copolymers is described in U.S. Patent No. 4,870,148 to RB Kunststoffpatent-Verwertungs AG and Belland AG, both of Switzerland, issued September 26, 1989, which is herein incorporated by reference. Such copolymers are commercially available from Belland AG, and the most preferred copolymer is available as product code "GBC 2620".

The ethylene/acrylic acid copolymers are high melt index dispersible polymers that are typically 20 percent acrylic acid by weight and 300 to 1300 in melt index. They are dispersible in alkaline water without emulsifiers, but are water-resistant in the acid form. Other ethylene/ acrylic acid copolymers which are available contain 3 to 9.5 percent acrylic acid by weight and have a melt index from 2.5 to 13.5. Such copolymers are commercially available from Dow Chemical Company, Midland, Michigan under the tradename PRIMACOR®, with the most preferred copolymers being those with product code numbers ranging from "5980" to "5990". They are produced by the free radical, high pressure copolymerization of ethylene and acrylic acid in a process similar to that used for producing low-density polyethylene.

Ethylene/methacrylic acid copolymers are commercially available from E.I. du Pont, Wilmington, Delaware, under the tradename NUCREL®, with grades ranging from 4 to 12 percent methacrylic acid by weight and from 2.5 to 13.5 in melt index.

Blends of the two copolymers can be prepared by mixing the desired weight ratio of the copolymer pellets and blending them using any standard equipment commonly used for blending thermoplastic polymers under conditions of heat and high shear. These include the Banbury® type of intensive production mixer (Farrel Corp., Ansonia, CT) and both single- and twin-screw compounding extruders, which can utilize high-shear mixing screws, co-rotating kneading blocks, etc.

In addition to blends containing the two above-mentioned copolymers, other components can be added to further enhance the properties of the resulting material. For example, polyethylene glycol can be added to lower the melt viscosity of these copolymers to a range suitable for meltblown or meltsprayed nonwovens and also improve the flexibility of the resulting webs. The amount of polyethylene glycol added to the copolymer blend can be from about 5 to about 20 weight percent, based on the total weight of the final blend, and a preferred range is from about 10 to about 15 weight percent. Suitable polyethylene glycols are available commercially from Union Carbide Corporation, Tarrytown, New Jersey, under the tradename CARBOWAX®; most suitable are product code numbers "3350" and "8000."

Polyethylene can also be added to blends containing the above-mentioned copolymers to improve the softness of the resulting nonwoven webs. The amount of polyethylene added to the copolymer blend can be from about 5 to about 15 weight percent, based on the total weight of the blend. The polyethylene grade must be selected so that the final blend has a melt index suitable for the nonwoven process to be used. Suitable fiber grade polyethylenes are available commercially from Dow Chemical Company, Midland, Michigan, under the tradename ASPUN®. Product code number "6811" is most suitable for blends for spunbond nonwovens and product code numbers "6806" and "6814" are most suitable for blends for meltblown or meltsprayed nonwovens.

Still further improvements to the properties of the webs of this invention, particularly films, can be made by adding certain fillers such as fumed silica, calcium carbonate or talc. Various particulate fillers have been shown to reduce blocking, noise and gloss in the films. Such materials can be added in amounts of from about 2 percent to about 20 weight percent, based on the total weight of the blend. Processing characteristics of the blends for both films and nonwovens can be improved by the incorporation of lubricants or slip agents into the blends. Additives of other types normally used in polymer blends can also be incorporated to provide specific properties as needed, such as antistatic agents, pigments or other colorants, and the like. All of these additive types are generally used in small amounts, usually less than 5 percent.

Films of the two copolymers can be prepared by extrusion of the blend through a linear film die, allowing the film to attenuate under its own weight into a nip between two chilled rolls. Alternatively, an annular die can be used to produce a polymeric tube, which can be attenuated by an air stream to form a film "bubble."

Nonwoven webs of the two copolymers can be prepared by extrusion of the blend through a plurality of capillaries, producing a series of filaments. These filaments can be quenched and then attenuated into fibers by an accelerating gas stream. The fibers can be collected on a moving surface, where they are deposited by the gas stream in a random fashion. Passing the resulting batt through a pair of heated rolls bonds the fibers together into an integral web. Alternatively, a hot gas stream may be used to attenuate and break the filaments in the molten state. These discontinuous fibers can be collected on a moving surface, where they will lay down in a random, entangled manner, producing an integral web. Suitable nonwoven webs include, without limitation, meltblown webs, spunbonded webs, and coform webs (meltblown webs in which a second fiber source, such as cellulose fibers, is blown into the primary meltblown fiber stream prior to deposition onto the collecting surface). All of such webs are known in the nonwovens art.

### EXAMPLES

### Comparative Example 1: Preparation of Copolymer Blend

A blend containing 80 percent by weight of Belland's blended copolymer product designated GBC 2620 WB was prepared. This product is reported to be a copolymer of ethyl acrylate and methacrylic acid in a ratio of 4:1 by weight, respectively. The blended product is reported to contain 1 percent by weight of titanium dioxide and 3 percent by weight of Hostalube® FA-1 (Hoechst Celanese, mixed amide of ethylenediamine with palmitic and stearic acids).

To prepare the blend of interest, this product was compounded with 18 percent by weight PRIMACOR® 5990 (Dow, ethylene-acrylic acid copolymer) and 2 percent by weight of Slip-Quick® (Synthetic Products Co., fatty amide slip agent). The ingredients were first mixed thoroughly by means of a drum tumbler, and then transferred to an Accurate® volumetric feeder. The mixture was metered into a Werner & Pfleiderer 30-mm twin-screw compounding extruder for blending. Extruder zones 1 through 6 were set with the following temperature profile (in °C): 100, 101, 110, 121, 140, 145; with the lowest temperature being at the feed zone adjacent to the throat of the extruder where the mixture enters and the highest temperature being at the die from which the molten blend exits.

The extruder screw configuration used was a vented, two-stage mixing screw combination of a type typically used for preparing polyolefin blends. Extruder throughput was maintained at about 22.7 kg/hour (ca. 50 pounds/hour). The molten blend was taken off from a four-strand die into a water bath with a dip length of six feet, then through two air knives to remove as much water as possible. The pellets were ejected from a rotating-knife pelletizer directly into a spin dryer to remove the remaining surface moisture before screening and packaging.

### Comparative Example 2: Preparation of Copolymer Blend Film

A copolymer blend containing 80% GBC 2620 WB, 10% PRIMACOR ® 5990, and 10% Microtuff® F (Pfizer, surface-treated talc) was blended as in Example 1. The polymer blend was then processed at a rate of 52.21 kg/hour (115 pounds/hour) through a single-screw extruder with an 8.89 cm (3.5 inch) diameter, internally-cooled screw (3:1 compression ratio, 24:1 L/D). A flat temperature profile of 125.7°C (260°F) was used in all extruder zones, transfer piping and in the film die. Film was produced using a 15.24 cm (six-inch) diameter annular die with a die gap of 0.107 cm (0.042 inches). A bubble was generated with a diameter of 35.56-45.72 cm (14-18 inches), producing film that varied from 0.0028-0.0036 cm (0.0011 to 0.0014 inches) in gauge. The film was collected at 25.2 meters per minute (84 feet per minute).

Ultimate tensile strengths were determined to be 1868 grams/2.54 cm (inch) at 220% elongation (machine direction) and 1400 grams/2.54 cm (inch) at 270% elongation (cross direction). A similarly-prepared film of 100% GBC 2620 WB with the same gauge of 0.0028 cm (0.0011 inch) had ultimate tensile strengths of 2647 grams/2.54 cm (inch) at 224% elongation (machine direction) and 1274 grams/2.54 cm (inch) at 365% elongation (cross direction). The films prepared from the described blend show less isotropy, less tendency to block, and better moisture resistance than the film prepared from GBC 2620 WB alone.

### Comparative Example 3: Preparation of Copolymer Blend Nonwoven Web

A copolymer blend containing 70% GBC 2620 WB, 28% PRIMACOR ® 5990, and 2% Slip-Quick® was prepared as in Example 1. A nonwoven web was prepared from this blend by means of a spunbond process. The polymer blend pellets were introduced into a single-screw extruder having a screw configuration of a type normally used for extrusion of polyolefins. The molten polymer, at a melt temperature of 171.11°C (340°F), was conveyed from the extruder through a metering pump to a fiber-spinning die. Molten polymer strands exiting the die were quenched, attenuated and formed into a web by controlled streams of high-velocity air. The resulting web was carried by a forming wire through compaction and bonding sections to form a point-bonded spunbond nonwoven.

Webs formed in this manner are dimensionally stable (less than 5% shrinkage) and possess acceptable drape, flexibility and softness. Webs prepared by the same process from GBC 2620 with no additives suffered severe shrinkage (up to 50%), distortion, and increasing rigidity over several weeks. Moisture was found to accelerate these changes.

### Example 4: Preparation and Processing of Copolymer Blend With Polyethylene Glycol

A copolymer blend containing 80% GBC 2630 AA (a 4.3:1 ethyl acrylate/methacrylic acid copolymer with no additives), 10% PRIMACOR® 5990, and 10% CARBOWAX® 3350 (Union Carbide, polyethylene glycol of molecular weight 3000-3700) was prepared using a 3/4" single-screw compounding extruder with a single mixing section and L/D of 26:1. Strands were generated through a dual-strand die at 160°C (320°F), allowed to air cool, and pelletized.

This blend was processed through a meltblown apparatus using a 1.91 cm (3/4") single-screw extruder with an L/D ratio of 26:1. The melt was metered into a meltblown die containing 14 capillaries in a 5.08 cm (2") width with a capillary diameter of 0.0368 cm (0.0145 inch). The extruder zones 1-3 were set for the following temperature profile (in °C): 57.2, 123.8, 171.1 (in °F: 136, 257, 342). The die and air were heated to 223.89°C (435°F). Fibers were produced using this apparatus and polymer blend with fiber diameters ranging from 0.01 to 0.04 mm. The fibers were laid down in a random manner on a screen, forming a meltblown web. The level of interfiber adhesion was high, and no further bonding was needed to maintain integrity in the web.

### Example 5: Preparation of Polymer Blend and Nonwoven Web

A copolymer blend containing 80% GBC 2630 AA (a 4.3:1 ethyl acrylate/methacrylic acid copolymer with no additives), and 20% CARBOWAX® 3350 (Union Carbide, polyethylene glycol of molecular weight 3000-3700) was prepared using a 1.91 cm (3/4") single-screw compounding extruder with a single mixing section and L/D of 26:1. Strands were generated through a dual-strand die at 160°C, allowed to air cool, and pelletized.

Intrinsic viscosity of this blend was measured using a capillary rheometer with a capillary diameter of 0.0513 cm (0.0202 inches) and L/D ratio of 4.89. The viscosity of the blend was 410 poise at 170°C and 1075s^{.1}. The unblended ethyl acrylate/methacrylic acid copolymer had a viscosity of 1178 poise under the same conditions. It was found that the blended polymers increased in viscosity over time when heated to 204.4°C (400°F), apparently due to a reaction in which the poly(ethylene glycol) grafts to the ethyl acrylate/methacrylic acid copolymer.

This blend was processed through a heated piston apparatus to force the molten blend through a 1 mm orifice at a temperature of 196.1°C (385°F). The filament formed was attenuated using hot air and deposited randomly on a moving collection wire, forming a web with some integrity. This polymer blend and forming method can be used to produce composite structures (coforms) suitable for a variety of personal care absorbent products. The composite is soluble and dispersible on immersion in a basic solution.

As a summary of the above, with respect to the above described embodiments and examples, the present invention provides an absorbent article comprising a polymeric web comprising a blend of from 60 to 95 weight percent of a copolymer of a (meth)acrylate ester and (meth)acrylic acid and from 5 to 50 weight percent of polyethylene glycol. The (meth)acrylate ester is preferably ethyl acrylate. The (meth)acrylic acid is preferably methacrylic acid. The copolymer of ethyl acrylate and methacrylic acid comprises preferably about 80 weight percent ethyl acrylate moieties and preferably about 20 weight percent methacrylic acid moieties. The web comprises preferably from 70 to 90 weight percent of a copolymer of ethyl acrylate and methacrylic acid and preferably from 5 to 30 weight percent of polyethylene glycol. More preferably, the web comprises about 80 weight percent of a copolymer of ethyl acrylate and methacrylic acid and about 20 weight percent polyethylene glycol. The web can be a nonwoven web.

The composition of matter comprises a polymeric blend of from 60 to 95 weight percent of a copolymer of a (meth)acrylate ester and (meth)acrylic acid and from 5 to 30 weight percent polyethylene glycol. The (meth)acrylate ester in the composition is preferably ethyl acrylate. The (meth)acrylic acid is preferably methacrylic acid. Advantageously, the copolymer of ethyl acrylate and methacrylic acid comprises about 80 weight percent ethyl acrylate moieties and about 20 weight percent methacrylic acid moieties. Preferably, the composition comprises from 70 to 90 weight percent of a copolymer of ethyl acrylate and methacrylic acid and from 5 to 30 weight percent of polyethylene glycol, and more preferably, the composition comprises about 80 weight percent of a copolymer of ethyl acrylate and methacrylic acid and about 20 weight percent polyethylene glycol.

The present invention provides an absorbent article having an outer cover, an absorbent core and an inner liner, wherein said absorbent core comprises a composite structure comprising the polymeric web. The absorbent article is preferably a diaper or a sanitary napkin.

Related to, but not forming part of the present invention is a composition of matter comprising a blend of from 50 to 90 weight percent of a copolymer of a (meth)acrylate ester and (meth)acrylic acid and from 10 to 50 weight percent of a copolymer of ethylene and (meth)acrylic acid. The composition of matter may further comprise from 5 to 15 weight percent polyethylene. Preferably, the (meth)acrylate ester is ethyl acrylate. The (meth)acrylic acid of the ethyl acrylate/(meth)acrylic acid copolymer is preferably methacrylic acid. Advantageously, the (meth)acrylic acid of the ethylene/(meth)acrylic acid copolymer is acrylic acid. The copolymer of ethyl acrylate and methacrylic acid may comprise about 80 weight percent ethyl acrylate moieties and about 20 weight percent methacrylic acid moieties. The copolymer of ethylene and acrylic acid can comprise about 80 weight percent ethylene moieties and about 20 weight percent acrylic acid moieties. The composition advantageously comprises from 70 to 90 weight percent, preferably from 80 to 90 weight percent, of a copolymer of ethyl acrylate and methacrylic acid and from 10 to 30 weight percent, preferably from 10 to 20 weight percent, of a copolymer of ethylene and acrylic acid.

Related to, but not forming part of the present invention is a polymeric web comprising a blend of from 50 to 90 weight percent of a copolymer of a (meth)acrylate ester and (meth)acrylic acid and from 10 to 50 weight percent of a copolymer of ethylene and (meth)acrylic acid. The web may further comprise from 5 to 15 weight percent polyethylene. The (meth)acrylate ester is preferably ethyl acrylate. The (meth)acrylic acid of the ethyl acrylate/(meth)acrylic acid copolymer is preferably methacrylic acid. Advantageously, the (meth)acrylic acid of the copolymer of ethylene and (meth)acrylic acid is acrylic acid. Preferably, the copolymer of ethyl acrylate and methacrylic acid comprises about 80 weight percent ethyl acrylate moieties and about 20 weight percent methacrylic acid moieties. Preferably, the copolymer of ethylene and acrylic acid comprises about 80 weight percent ethylene moieties and about 20 weight percent acrylic acid moieties. The web advantageously comprises from 70 to 90 weight percent, preferably from 80 to 90 weight percent, of a copolymer of ethyl acrylate and methacrylic acid and from 10 to 30 weight percent, preferably from 10 to 20 weight percent, of a copolymer of ethylene and acrylic acid.

Related to, but not forming part of the present invention is a film comprising a blend of from 65 to 90 weight percent of a copolymer of ethyl acrylate and methacrylic acid and from 10 to 20 weight percent of a copolymer of ethylene and acrylic acid, wherein said copolymer of ethyl acrylate and methacrylic acid comprises about 80 weight percent ethyl acrylate moieties and about 20 weight percent methacrylic acid moieties and wherein said copolymer of ethylene and acrylic acid contains about 80 weight percent ethylene moieties and about 20 weight percent acrylic acid moieties. The film may further comprise from 5 to 15 weight percent polyethylene.

Related to, but not forming part of the present invention is a nonwoven web comprising a blend of from 50 to 90 weight percent, preferably 70 to 80 weight percent, of a copolymer of ethyl acrylate and methacrylic acid and from 10 to 50 weight percent, preferably from 15 to 30 weight percent, of a copolymer of ethylene and acrylic acid, wherein said copolymer of ethyl acrylate and methacrylic acid comprises about 80 weight percent ethyl acrylate moieties and about 10 weight percent methacrylic acid moieties and wherein said copolymer of ethylene and acrylic acid comprises about 80 weight percent ethylene moieties and about 20 weight percent acrylic acid moieties. The nonwoven web may further comprise from 5 to 15 weight percent polyethylene. Advantageously, the nonwoven web comprises from 5 to 20, preferably from 10 to 15, weight percent of polyethylene glycol. Preferably, the nonwoven web comprises a blend of about 80 weight percent of the copolymer of ethyl acrylate and methacrylic acid, about 10 weight percent of the copolymer of ethylene and acrylic acid, and about 10 weight percent polyethylene glycol. The nonwoven web can comprise from 5 to 15 weight percent polycaprolactone. The web is preferably a meltblown web or a spunbonded web or a coform web.

The present invention provides an absorbent article comprising an outer cover, an absorbent core and an inner liner, wherein either of said outer cover or said inner liner is a nonwoven web as described above. The absorbent article can be a diaper or a sanitary napkin.

## Claims

1. An absorbent article having an outer cover, an absorbent core and an inner liner wherein any or all of said outer cover, absorbent core and inner liner comprise a nonwoven web or film prepared from a composition comprising a polymeric blend of from 60 to 95 weight percent of a copolymer of a (meth)acrylate ester and (meth)arylic acid and from 5 to 30 percent polyethylene glycol, wherein cross linked copolymers of a (meth)acrylate ester and (meth)acrylic acid are excluded.

2. The absorbent article of claim 1 wherein the (meth)acrylate ester is ethyl acrylate.

3. The absorbent article of claim 1 or 2 wherein (meth)acrylic acid is methacrylic acid.

4. The absorbent article of one of the preceding claims wherein the copolymer of ethyl acrylate and methacrylic acid comprises about 80 weight percent ethyl acrylate moieties and about 20 weight percent methacrylic acid moieties.

5. The absorbent article of one of the preceding claims wherein said nonwoven web or film comprises 70 to 90 weight percent of a copolymer of ethyl acrylate and methacrylic acid and from 5 to 30 weight percent of polyethylen glycol.

6. The absorbent article of claim 5 wherein said nonwoven web or film comprises about 80 weight percent of a copolymer of ethyl acrylate and methacrylic acid and about 20 weight percent polyethylen glycol.

7. The absorbent article of claim any one of the preceding claims wherein the absorbent article is a diaper or a sanitary napkin.

## Patentansprüche

1. Saugfähiger Artikel mit einer Außenhülle, einem Saugkern und einer Inneneinlage, wobei ein Teil oder alle Teile ausgewählt aus der Außenhülle, dem Saugkern und der Inneneinlage eine Vliesbahn oder einen Film aufweisen, hergestellt aus einer Zusammensetzung, umfassend eine Polymermischung aus 60 bis 95 Gew.% eines Copolymers aus einem (Meth)acrylatester und (Meth)acrylsäure und 5 bis 30 Prozent Polyethylenglycol, wobei vernetzte Copolymere eines (Meth)acrylatesters und (Meth)acrylsäure ausgeschlossen sind.

2. Saugfähiger Artikel gemäß Anspruch 1, wobei es sich bei dem (Meth)acrylatester um Ethylacrylat handelt.

3. Saugfähiger Artikel gemäß Anspruch 1 oder 2, wobei es sich bei der (Meth)acrylsäure um Methacrylsäure handelt.

4. Saugfähiger Artikel gemäß einem der vorhergehenden Ansprüche, bei dem das Copolymer von Ethylacrylat und Methacrylsäure etwa 80 Gew.% Ethylacrylateinheiten und etwa 20 Gew.% Methacrylsäureeinheiten enthält.

5. Saugfähiger Artikel gemäß einem der vorhergehenden Ansprüche, bei dem die Vliesbahn oder der Film 70 bis 90 Gew.% eines Copolymers von Ethylacrylat und Methacrylsäure und 5 bis 30 Gew.% von Polyethylengylcol umfaßt.

6. Saugfähiger Artikel gemäß Anspruch 5, bei dem die Vliesbahn oder der Film etwa 80 Gew.% eines Copolymers von Ethylacrylat und Methacrylsäure und etwa 20 Gew.% Polyethylengylcol umfaßt.

7. Saugfähiger Artikel gemäß einem der vorhergehenden Ansprüche, wobei es sich bei dem saugfähigen Artikel um eine Windel oder eine Damenbinde handelt.

## Revendications

1. Article absorbant ayant une enveloppe extérieure, un noyau absorbant et une doublure intérieure, dans lequel l'un quelconque, ou tous, desdits enveloppe extérieure, noyau absorbant et doublure intérieure comprend une nappe non tissée ou un film préparé à partir d'une composition comprenant un mélange polymère de 60 à 95% en poids d'un copolymère d'un ester (méth)acrylique et d'acide (méth)acrylique et de 5 à 30% de polyéthylène glycol, les copolymères réticulés d'un ester (méth)acrylique et d'un acide (méth)acrylique étant exclus.

2. Article absorbant selon la revendication 1, dans lequel l'ester (méth)acrylique est l'acrylate d'éthyle.

3. Article absorbant selon la revendication 1 ou 2, dans lequel l'acide (méth)acrylique est l'acide méthacrylique.

4. Article absorbant selon l'une des revendications précédentes, dans lequel le copolymère d'acrylate d'éthyle et d'acide méthacrylique comprend environ 80% en poids de motifs acrylate d'éthyle et environ 20% en poids de motifs acide méthacrylique.

5. Article absorbant selon l'une des revendications précédentes, dans lequel ladite nappe non tissée ou ledit film comprend 70 à 90% en poids d'un copolymère d'acrylate d'éthyle et d'acide méthacrylique et de 5 à 30% en poids de polyéthylène glycol.

6. Article absorbant selon la revendication 5, dans lequel ladite nappe non tissée ou ledit film comprend environ 80% en poids d'un copolymère d'acrylate d'éthyle et d'acide méthacrylique et environ 20% en poids de polyéthylène glycol.

7. Article absorbant selon l'une quelconque des revendications précédentes, qui est un change pour bébés ou une serviette hygiénique.
